# DEMANDE DE BREVET EUROPEEN

(11) **EP 2 805 685 A1**
(43) Date de publication de la demande: **26.11.2014**
(21) Numéro de dépôt: 14168473.8
(22) Date de dépôt: 15.05.2014
(51) Int. Cl.: A61B 19/00, G06F 3/01

(54) **Système d'éclairage médical, notamment d'éclairage operatoire, et procédé de commande d'un tel système d'éclairage**

(30) Priorité: 24.05.2013 FR 1354675
(71) Demandeur: Surgiris, 59170 Croix (FR)
(72) Inventeur: Micucci, Daniel, 7711 Dottignies (BE); Papin, Denis, 59970 Fresnes Sur Escaut (FR)
(74) Mandataire: Balesta, Pierre

(57) **Abrégé**

La présente invention concerne un système d'éclairage médical (1), notamment d'éclairage opératoire, comprenant un ensemble de sources lumineuses (2) pour éclairer une surface déterminée et présentant une ou plusieurs propriétés optiques modifiables, et un dispositif de commande (6) de l'ensemble de sources lumineuses (2), pour modifier ladite ou lesdites propriétés optiques en fonction d'une commande d'un utilisateur.

En particulier, le dispositif de commande (6) est un dispositif de commande à interface gestuelle comprenant :
- deux capteurs (7, 8) montés à distance l'un de l'autre, chaque capteur délivrant un signal correspondant à une zone de détection et les zones de détection des deux capteurs (7, 8) comprenant une zone de détection commune pour détecter une commande gestuelle de l'utilisateur, et
- un moyen d'analyse, recevant en entrée les signaux issus des deux capteurs (7, 8) et configuré pour modifier ladite ou lesdites propriétés optiques en fonction de ladite commande gestuelle.

L'invention concerne également le procédé de commande d'un tel système d'éclairage (1).

## Description

### Arrière-plan de l'invention

La présente invention concerne un système d'éclairage médical, notamment d'éclairage opératoire, du type connu sous le nom de Scialytique, émettant un faisceau lumineux dirigé au-dessus d'un champ opératoire.

Les systèmes d'éclairage médical connus comprennent un ensemble de sources lumineuses montées à distance du champ opératoire de telle façon à éclairer, avec une intensité lumineuse maximale, une partie du champ opératoire, dite champ lumineux. Le réglage du champ lumineux, notamment sa focalisation, est effectué soit par le chirurgien lui-même au moyen d'une poignée stérilisante montée sur le corps des sources lumineuses, soit par un auxiliaire sur l'ordre du chirurgien.

Cependant, dans le cas d'une manipulation par le chirurgien, la stérilité de la poignée doit être assurée de façon totale durant toute la durée de l'opération. De plus, pour saisir la poignée, le chirurgien est amené à regarder vers le corps des sources lumineuses, c'est-à-dire dans le faisceau lumineux, ce qui provoque son éblouissement.

Dans le cas d'une manipulation par un auxiliaire, le réglage précis du champ lumineux est rendu difficile par la position dudit auxiliaire qui se trouve à distance du chirurgien, et donc à distance du champ lumineux à régler. Le réglage s'effectue donc suivant des ordres donnés par le chirurgien, ce qui le rend plus long et moins précis.

### Objet et résumé de l'invention

La présente invention vise à résoudre les différents problèmes techniques énoncés précédemment. En particulier, la présente invention vise à proposer un système d'éclairage médical permettant un réglage plus souple et précis pour l'utilisateur. La présente invention vise également à proposer un système d'éclairage médical limitant les contraintes de stérilité pour l'utilisateur.

Ainsi, selon un aspect, il est proposé un système d'éclairage médical, notamment d'éclairage opératoire, comprenant :
- un ensemble de sources lumineuses pour éclairer une surface déterminée, par exemple un champ opératoire, et présentant une ou plusieurs propriétés optiques modifiables,
- un dispositif de commande de l'ensemble de sources lumineuses, pour modifier ladite ou lesdites propriétés optiques en fonction d'une commande d'un utilisateur.

En particulier, le dispositif de commande est un dispositif de commande à interface gestuelle comprenant :
- deux capteurs montés à distance l'un de l'autre, chaque capteur délivrant un signal correspondant à une zone de détection et les zones de détection des deux capteurs comprenant une zone de détection commune pour détecter une commande gestuelle de l'utilisateur, et
- un moyen d'analyse, recevant en entrée les signaux issus des deux capteurs et configuré pour modifier ladite ou lesdites propriétés optiques en fonction de ladite commande gestuelle.

Grâce à l'analyse bidimensionnelle de la zone de détection commune, le système d'éclairage peut être commandé facilement et de manière précise par le chirurgien lui-même. Il n'est notamment plus nécessaire pour lui de regarder le système placé au-dessus de lui, et d'être ébloui pendant l'opération. Il peut notamment modifier la focalisation de l'éclairage, la luminosité, la couleur, etc, via le contrôle des sources lumineuses, ou via le contrôle d'éléments optiques, tels que des filtres ou des dioptres, pouvant être positionnés et déplacés électriquement dans le faisceau lumineux des sources lumineuses.

Par ailleurs, l'utilisation de deux capteurs distants et présentant une zone de détection commune, c'est-à-dire de deux capteurs montés en relation stéréoscopique, permet d'analyser une commande gestuelle en trois dimensions, dans l'espace, et pas seulement dans un plan. Cela permet d'une part de disposer d'une gamme de commandes gestuelles plus importante, et d'autre part de distinguer les gestes effectués lors du travail de l'utilisateur, par exemple les gestes effectués au niveau du champ opératoire par le chirurgien, des gestes effectués par l'utilisateur pour commander le système d'éclairage.

On obtient un système d'éclairage médical, en particulier d'éclairage opératoire (c'est-à-dire sans ombres portées), facile à commander par le chirurgien et sans contrainte de stérilisation.

Préférentiellement, un moyen d'analyse reçoit en entrée les signaux issus des deux capteurs et est configuré pour modifier ladite ou lesdites propriétés optiques en fonction de ladite commande gestuelle lorsque la commande gestuelle est effectuée dans la zone de détection commune, à une distance de la surface déterminée supérieure à une valeur déterminée.

En particulier, le moyen d'analyse est configuré pour ne pas modifier ladite ou lesdites propriétés optiques en fonction de ladite commande gestuelle lorsque la commande gestuelle est effectuée dans la zone de détection commune, à une distance de la surface déterminée inférieure à une valeur déterminée.

Autrement dit, le moyen d'analyse est configuré pour modifier ladite ou lesdites propriétés optiques en fonction de commandes gestuelles effectuées uniquement à distance de la surface déterminée.

Préférentiellement, les deux capteurs sont des caméras.

Préférentiellement, l'ensemble de sources lumineuses comprend un module central comportant plusieurs sources lumineuses, et un ou plusieurs modules auxiliaires, comportant plusieurs sources lumineuses. Les modules auxiliaires peuvent être montés fixes autour du module central, par exemple selon une configuration en nid d'abeille, ou articulés mécaniquement par rapport au module central de manière à pouvoir être inclinés par rapport à celui-ci.

Préférentiellement, le moyen d'analyse comprend un moyen d'identification de la commande gestuelle recevant en entrées les signaux issus des deux capteurs et configuré pour délivrer un signal de commande.

Le moyen d'identification de la commande gestuelle peut comprendre :
- un moyen de reconnaissance du geste recevant en entrées les signaux issus des deux capteurs et configuré pour délivrer un ou des signaux identifiant un geste de l'utilisateur dans la zone commune des deux capteurs, et
- un moyen de sélection de la commande recevant en entrée le ou les signaux délivrés par le moyen de reconnaissance du geste, et configuré pour délivrer le signal de commande correspondant au geste identifié par le moyen de reconnaissance du geste.

Ainsi, l'identification de la commande gestuelle est effectuée en deux étapes : d'une part, un moyen de reconnaissance du geste analyse les signaux issus de deux capteurs afin d'identifier précisément le geste effectué dans la zone commune de détection, puis le résultat du moyen de reconnaissance du geste est envoyé à un moyen de sélection de la commande qui va associer au geste identifié précédemment une commande prédéfinie. Plus l'identification du geste est précise, plus l'identification de la commande associée sera facile et rapide.

Préférentiellement, le moyen de reconnaissance du geste comprend :
- un moyen de perception global pour identifier les éléments présents dans la zone de détection commune, recevant en entrées les signaux des deux capteurs et configuré pour délivrer un ou des premiers signaux de reconnaissance du geste, de préférence sous forme d'histogrammes spatio-temporels ;
- un moyen de perception dynamique pour identifier des mouvements dans la zone de détection commune, recevant en entrées les signaux des deux capteurs et configuré pour délivrer un ou des deuxièmes signaux de reconnaissance du geste, de préférence sous forme d'histogrammes spatio-temporels, et
- un moyen de perception structurelle pour identifier des formes dans la zone de détection commune, recevant en entrées les signaux des deux capteurs et configuré pour délivrer un ou des troisièmes signaux de reconnaissance du geste, de préférence sous forme d'histogrammes spatio-temporels.

L'utilisation de moyens de perception délivrant des signaux sous forme d'histogrammes spatio-temporels permet d'obtenir un traitement en temps réel rapide et efficace des signaux issus des deux capteurs. Les moyens de perception (globale, dynamique, structurelle) permettent notamment de déterminer les informations pertinentes des signaux issus des deux capteurs, et de les transmettre, par exemple sous forme d'histogrammes spatio-temporels, au moyen de sélection de la commande. On limite ainsi la quantité de données transmise au moyen de sélection de la commande, ce qui permet un traitement plus rapide et plus efficace par le moyen de sélection de la commande.

Préférentiellement, le moyen de sélection de la commande comprend un moyen d'activation pour autoriser l'identification de la commande lorsque la commande gestuelle est effectuée dans la zone de détection commune, à une distance de la surface déterminée supérieure à une valeur déterminée. Le moyen d'activation permet au dispositif de commande de distinguer les gestes effectués par l'utilisateur au niveau de la surface déterminée, de ceux effectués en hauteur qui sont attribués à la commande gestuelle. Le moyen d'activation définit ainsi une zone d'activation en-dehors de laquelle les gestes de l'utilisateur ne sont pas pris en considération. Seuls les gestes effectués dans la zone d'activation sont pris en compte et identifiés à une commande.

Le moyen d'analyse peut également comprendre un moyen de contrôle, recevant en entrée le signal de commande délivré par le moyen d'identification de la commande gestuelle et configuré pour modifier ladite ou lesdites propriétés optiques en fonction dudit signal de commande. Le moyen de contrôle permet de traduire la commande en signaux de contrôle modifiant les propriétés optiques de l'ensemble de sources lumineuses. Le moyen de contrôle peut ainsi alimenter ou cesser l'alimentation électrique de certaines sources lumineuses. Il peut également commander le déplacement d'un dioptre placé entre les sources lumineuses et le champ opératoire, afin de changer la focalisation du faisceau. Il peut également ajouter ou supprimer des filtres, ou bien encore alimenter ou cesser d'alimenter des sources lumineuses spécifiques, par exemple présentant un spectre d'émission particulier.

Préférentiellement, le dispositif de commande comprend également un moyen d'information sonore, pour émettre un signal sonore audible par l'utilisateur indiquant, par exemple, la détection d'une commande gestuelle ou l'attente d'une commande gestuelle. Le moyen d'information sonore permet de communiquer avec l'utilisateur sur la prise en compte de ses commandes gestuelles. Grâce à ce moyen sonore, l'utilisateur n'est pas obligé de regarder un écran ou des lumières de contrôle pour vérifier que ses commandes ont bien été analysées et prises en compte : un simple signal sonore l'informe de sorte qu'il peut, en absence d'un tel signal sonore, recommencer sa commande gestuelle pour qu'elle soit prise en compte.

Préférentiellement, le moyen d'information sonore est configuré pour émettre un signal sonore audible par l'utilisateur lorsque le moyen d'activation autorise l'identification de la commande. Le moyen d'information sonore permet dans ce cas d'informer l'utilisateur qu'il se trouve dans la zone d'activation et que ses commandes gestuelles sont analysées. Ainsi, en l'absence d'un tel signal sonore, l'utilisateur peut savoir que ses gestes ne sont pas considérés par le dispositif de commande. A l'inverse, lorsque le signal sonore est émis, l'utilisateur sait qu'il peut effectuer la commande gestuelle et que celle-ci sera prise en compte.

Préférentiellement, le dispositif de commande est configuré pour modifier ladite ou lesdites propriétés optiques par modification individuelle ou groupée de l'intensité lumineuse de sources lumineuses. Dans ce cas, les modifications demandées par l'utilisateur sont contrôlées de manière électronique, sans déplacement d'élément du système d'éclairage. On obtient ainsi une réponse à la commande plus rapide, et un système d'éclairage plus simple grâce à l'absence de moteur de déplacement. De préférence, le système d'éclairage comprend un élément optique monté entre au moins une partie des sources lumineuses et la surface déterminée, la taille et/ou la forme de la surface déterminée étant modifiée par contrôle, individuel ou groupé, de l'intensité lumineuse émise par ladite au moins une partie des sources lumineuses.

Selon un autre aspect, l'invention concerne également un procédé de commande à interface gestuelle d'un système d'éclairage médical, notamment d'éclairage opératoire, le système d'éclairage médical comprenant un ensemble de sources lumineuses qui est configuré pour éclairer une surface déterminée et qui présente une ou plusieurs propriétés optiques modifiables. Le procédé comporte les étapes successives suivantes :
a) une étape de détection, dans une zone de détection commune à deux zones de détection différentes, d'une commande gestuelle d'un utilisateur, puis
b) une étape d'analyse de ladite commande gestuelle afin de modifier ladite ou lesdites propriétés optiques en fonction de ladite commande gestuelle.

Préférentiellement, l'étape d'analyse de ladite commande gestuelle modifie ladite ou lesdites propriétés optiques en fonction de ladite commande gestuelle lorsque la commande gestuelle est effectuée dans la zone de détection commune, à une distance de la surface déterminée supérieure à une valeur déterminée.

En particulier, l'étape d'analyse de ladite commande gestuelle ne modifie pas ladite ou lesdites propriétés optiques en fonction de ladite commande gestuelle lorsque la commande gestuelle est effectuée dans la zone de détection commune, à une distance de la surface déterminée inférieure à une valeur déterminée.

Autrement dit, l'étape d'analyse modifie ladite ou lesdites propriétés optiques en fonction de commandes gestuelles effectuées uniquement à distance de la surface déterminée.

Préférentiellement, l'étape d'analyse comporte :
b1) une étape d'identification de ladite commande gestuelle.

Préférentiellement, l'étape b1) d'identification de ladite commande gestuelle comprend :
b11) une étape de reconnaissance du geste, puis
b12) une étape de sélection de la commande correspondant au geste identifié à l'étape b11).

Préférentiellement, l'étape b11) de reconnaissance du geste comprend :
- une perception globale identifiant les éléments présents dans la zone de détection commune,
- une perception dynamique identifiant des mouvements dans la zone de détection commune, et
- une perception structurelle identifiant des formes dans la zone de détection commune.

Préférentiellement, l'étape de sélection de la commande comprend une étape d'activation autorisant l'identification de la commande lorsque la commande gestuelle est effectuée dans la zone de détection commune, à une distance de la surface déterminée supérieure à une valeur déterminée.

Préférentiellement, l'étape d'analyse comprend une étape de modification de ladite ou desdites propriétés optiques en fonction de ladite commande gestuelle.

Préférentiellement, le procédé comprend également une étape d'information sonore durant laquelle un signal sonore audible par l'utilisateur est émis pour indiquer, par exemple, la détection d'une commande gestuelle ou l'attente d'une commande gestuelle.

Préférentiellement, l'étape d'information sonore émet un signal sonore audible par l'utilisateur lorsque l'étape d'activation autorise l'identification de la commande.

### Brève description des dessins

L'invention et ses avantages seront mieux compris à la lecture de la description détaillée d'un mode de réalisation particulier, pris à titre d'exemple nullement limitatif et illustré par les dessins annexés sur lesquels :
- la figure **1** représente une partie d'un système d'éclairage médical selon l'invention comprenant un dispositif de commande à interface gestuelle, et
- la figure **2** est une représentation schématique des moyens fonctionnels du dispositif de commande à interface gestuelle.

### Description détaillée de l'invention

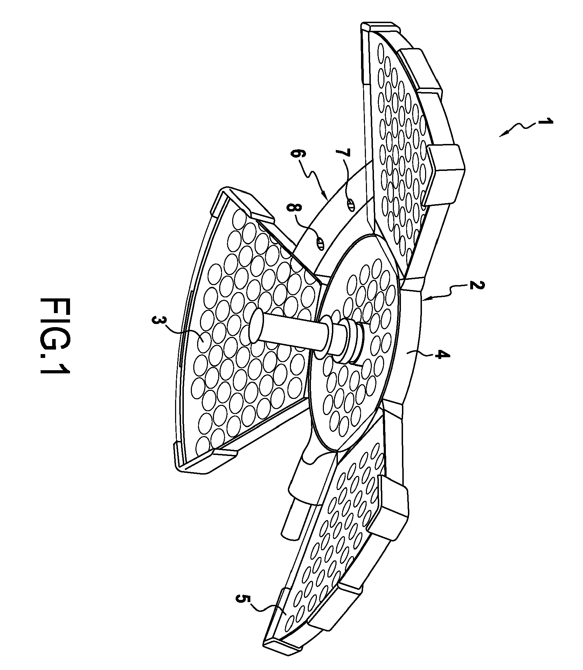

La figure 1 représente un exemple de mode de réalisation d'un système d'éclairage médical 1 selon l'invention. Le système d'éclairage médical 1 comprend notamment un ensemble 2 de sources lumineuses 3, de préférence des sources à flux modulable, par exemple de type LED. L'ensemble 2 comporte : un module central 4 et éventuellement des modules auxiliaires 5, par exemple au nombre de trois. Les modules auxiliaires 5 sont disposés autour du module central 4. Les modules auxiliaires 5 peuvent par exemple être régulièrement espacés autour du module central 4, par exemple à 120° les uns des autres, afin d'obtenir un éclairage uniforme dans toutes les directions. Ainsi, le système d'éclairage médical 1 peut être un système d'éclairage permettant d'éclairer une surface déterminée sans ombres portées, et être utilisé dans un bloc opératoire, pour une opération chirurgicale. Le système d'éclairage médical 1 sera alors nommé système d'éclairage opératoire.

De préférence, le système d'éclairage médical 1 comprend un dioptre spécifique monté entre au moins une partie des sources lumineuses 3 et la surface déterminée. Le dioptre spécifique peut comprendre différentes facettes inclinées en regard de sources lumineuses, permettant notamment de faire varier la taille, et éventuellement la forme, de la surface déterminée par contrôle, individuel ou groupé, de l'intensité lumineuse émise par ladite au moins une partie des sources lumineuses. Un tel dioptre est notamment décrit dans la demande EP 2 065 634. Dans ce cas, les modules auxiliaires peuvent être montés fixes sur le module central 4.

Dans la suite de la description, on considère que le système d'éclairage 1 est un système d'éclairage opératoire pour lequel l'utilisateur est un chirurgien et la surface déterminée est la surface éclairée du champ opératoire.

Le système d'éclairage médical 1 peut comprendre également un dispositif de déplacement (non-représenté). Le dispositif de déplacement peut comporter deux bras fixés l'un à l'autre manière pivotante. Un des bras peut être fixé à un mur ou au plafond, par exemple de manière pivotante, et l'autre bras peut être fixé à l'ensemble de sources lumineuses 2, par exemple de manière pivotante. Le dispositif de déplacement permet de déplacer et/ou d'orienter l'ensemble de sources lumineuses 2 de manière adéquate par rapport à la surface à éclairer.

Par ailleurs, les propriétés d'éclairage des sources lumineuses 3 peuvent être également amenées à changer, sur instructions du chirurgien, afin d'adapter la surface éclairée du champ opératoire, à la partie opérée. Ainsi, la surface éclairée peut être agrandie, rétrécie, plus ou moins éclairée, ou encore éclairée par une lumière présentant une température de couleur modifiée. Afin de changer les caractéristiques de la surface éclairée, et afin d'éviter de manipuler une poignée de commande stérile, le système d'éclairage médical 1 comprend un dispositif de commande 6, monté par exemple sur le module central 4 du système d'éclairage 1, et comportant notamment deux capteurs 7 et 8.

Les capteurs 7, 8, par exemple deux caméras, sont montés de préférence sur l'ensemble de sources lumineuses 2, et orientés comme les sources lumineuses, afin de faire face au champ opératoire et plus particulièrement à la surface éclairée au niveau de laquelle travaille le chirurgien. Les capteurs 7, 8 sont montés à distance l'un de l'autre, de manière à présenter des zones de détection distinctes mais avec une partie commune dite zone de détection commune. L'utilisation de deux capteurs pour détecter des éléments dans une zone de détection commune permet, par stéréoscopie, de déterminer la position des éléments en trois dimensions. Autrement dit, les capteurs 7, 8, montés en relation stéréoscopique, permettent au dispositif de commande 6 d'avoir une vision binoculaire de la surface éclairée.

Le dispositif de commande 6 permet à l'utilisateur, de commander le système d'éclairage 1, en particulier les propriétés optiques de l'ensemble des sources lumineuses 2, sans nécessiter de contact avec le système d'éclairage 1. Le dispositif de commande 6 permet notamment de détecter et d'interpréter les mouvements du chirurgien afin que ce dernier puisse modifier les propriétés optiques de l'ensemble des sources lumineuses 2 avec des gestes.

Par exemple, afin de distinguer d'une part les gestes de commande et d'autre part les gestes opératoires effectués par le chirurgien dans le cadre de l'opération, le dispositif de commande 6 peut détecter tous les mouvements effectués dans la zone de détection commune mais n'analyser que ceux effectués à une distance inférieure à une valeur déterminée des deux capteurs 7, 8. Par exemple, dans le cas d'une opération chirurgicale pour laquelle le système d'éclairage médical 1 est conçu pour éclairer de manière optimale une surface située à 1 mètre de son aplomb, le dispositif de commande 6 peut être configuré pour n'analyser que les mouvements effectués par le chirurgien à une distance inférieure à cinquante centimètres des capteurs 7, 8, c'est-à-dire les mouvements effectués volontairement par le chirurgien pour commander le système d'éclairage médical 1.

Une fois dans la zone d'analyse du dispositif de commande 6, le chirurgien peut alors modifier les propriétés optiques de l'ensemble de sources lumineuses avec des gestes prédéfinis. Par exemple, le chirurgien peut modifier le niveau d'éclairement du système d'éclairage 1 en déplaçant sa main, perpendiculairement à l'axe du bras, dans un plan parallèle à celui du module central 4. Un déplacement vers la droite peut augmenter le niveau d'éclairement et un déplacement vers la gauche le diminuer, ou inversement. De même, le chirurgien peut modifier la focalisation du système d'éclairage 1 en déplaçant sa main de haut en bas. Un déplacement vers le haut peut augmenter la focalisation et un déplacement vers le bas la diminuer, ou inversement. Enfin, le chirurgien peut modifier la température de couleur du système d'éclairage 1 en déplaçant sa main, dans l'axe du bras, dans un plan parallèle à celui du module central 4. Un déplacement vers l'avant peut augmenter la température de couleur tandis qu'un déplacement vers l'arrière peut le diminuer, ou inversement.

Pour chacune de ces commandes, la modification de la propriété optique peut avoir lieu à chaque mouvement du chirurgien. Alternativement, le dispositif de commande 6 peut être configuré pour augmenter ou diminuer graduellement la propriété optique concernée aussi longtemps que le chirurgien garde sa main dans la position de commande.

Chaque modification des propriétés optiques du système d'éclairage 1 peut être indiquée par un signal sonore permettant au chirurgien de savoir que sa commande a été prise en compte par le dispositif de commande 6. De même, lorsque le chirurgien place sa main dans la zone d'analyse du dispositif de commande 6, celui-ci peut émettre un signal sonore afin d'indiquer au chirurgien que les mouvements effectués seront considérés comme des gestes de commande.

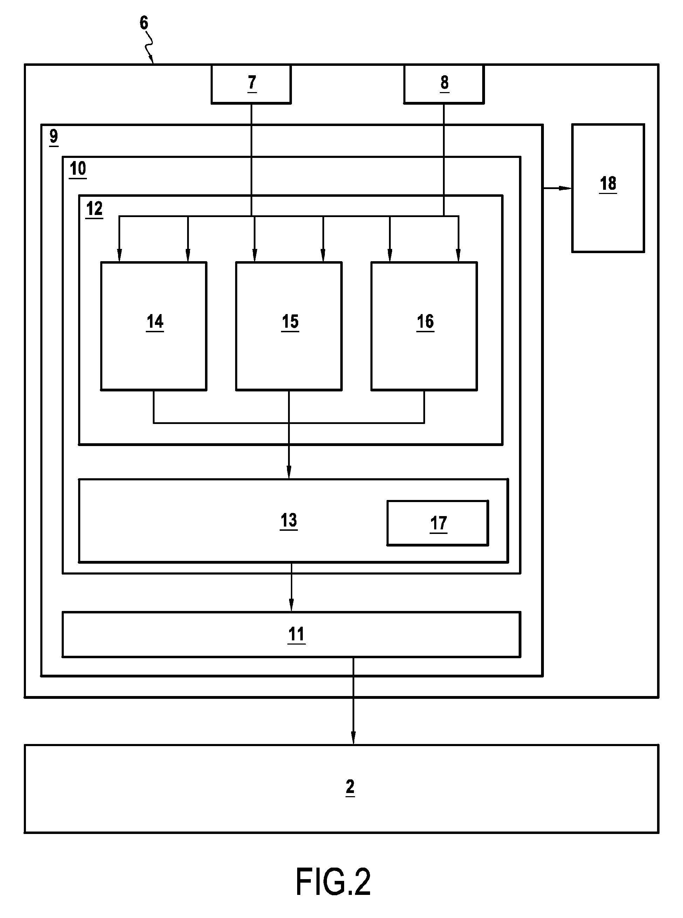

Une représentation schématique des moyens fonctionnels du dispositif de commande 6 est illustrée sur la figure 2.

Le dispositif de commande 6 comprend ainsi un moyen d'analyse 9 recevant en entrées les signaux des capteurs 7, 8, et fournissant, en sortie, des signaux de modifications des propriétés optiques de l'ensemble de sources lumineuses 2. Les signaux de modifications peuvent être des signaux d'alimentation électrique d'une ou plusieurs sources lumineuses, afin de les alimenter ou non selon la commande de l'utilisateur, ou bien encore des signaux modifiant, de manière individuelle ou groupée, la puissance d'alimentation d'une ou plusieurs sources lumineuses, afin d'obtenir, pour chaque source lumineuse, une intensité lumineuse déterminée comprise entre la valeur nulle (source lumineuse éteinte) et la valeur maximale. En particulier, avec le dioptre particulier mentionné plus haut, la taille et la forme de la surface éclairée du champ opératoire peuvent être ainsi contrôlées sans action mécanique du système d'éclairage, mais uniquement par contrôle, individuel ou groupé, de l'intensité lumineuse d'au moins une partie des sources lumineuses. Les commandes du chirurgien peuvent ainsi être exécutées plus rapidement, par contrôle électronique, et sans intervention de moteur de déplacement.

Le moyen d'analyse 9 comprend un moyen d'identification de la commande gestuelle 10, et un moyen de contrôle 11.

Le moyen d'identification de la commande gestuelle 10 reçoit en entrée les signaux des capteurs 7, 8 et est configuré pour identifier les commandes gestuelles effectuées par le chirurgien dans la zone de détection commune. Le moyen d'identification de la commande gestuelle 10 transmet alors un signal de commande correspondant à la commande gestuelle du chirurgien, au moyen de contrôle 11. Le moyen de contrôle 11 permet d'associer au signal de commande reçu, un signal de modification des propriétés optiques de l'ensemble des sources lumineuses 2.

Le moyen d'identification de la commande gestuelle 10 permet d'analyser les commandes gestuelles du chirurgien en deux étapes principales. Il comprend ainsi un moyen de reconnaissance du geste 12, qui permet d'identifier, dans les signaux issus des ceux capteurs 7, 8, les gestes effectués par le chirurgien, et un moyen de sélection de la commande 13, permettant d'associer aux gestes identifiés par le moyen de reconnaissance du geste 12, une commande déterminée.

Le moyen de reconnaissance du geste 12 reçoit en entrée les signaux des deux capteurs 7, 8, et est configuré pour délivrer un ou des signaux d'identification du geste au moyen de sélection de la commande 13. Le moyen de reconnaissance du geste 12 peut comprendre trois moyens de perception afin d'analyser les gestes effectués par le chirurgien : le moyen de perception globale 14, le moyen de perception dynamique 15 et le moyen de perception structurelle 16.

Le moyen de perception globale 14 reçoit en entrée les signaux des deux capteurs 7, 8 et est configuré pour délivrer un ou des premiers signaux d'identification du geste, de préférence sous forme d'histogrammes spatio-temporels. Le moyen de perception globale 14 permet, pour différents signaux successifs, de définir des valeurs permettant de représenter sous forme d'histogrammes les valeurs des signaux des capteurs 7, 8. Un tel dispositif et procédé est notamment décrit dans la demande de brevet FR 2 611 063.

Ainsi, le moyen de perception globale 14 permet d'identifier, à l'aide d'histogrammes, des éléments dans les signaux issus des capteurs 7, 8. De plus, comme le moyen de perception globale 14 reçoit les signaux des deux capteurs, il est également capable d'identifier les éléments présents dans ces signaux, en trois dimensions.

Le moyen de perception dynamique 15 reçoit en entrée les signaux des deux capteurs 7, 8 et est configuré pour délivrer un ou des deuxièmes signaux d'identification du geste, de préférence sous forme d'histogrammes spatio-temporels. Le moyen de perception dynamique 15 permet, pour différents signaux successifs, de détecter des variations de valeur temporelles et dans l'espace, et de les représenter sous forme d'histogrammes. Un tel dispositif et procédé est notamment décrit dans la demande de brevet FR 2 751 772.

Ainsi, le moyen de perception dynamique 15 permet d'identifier, à l'aide d'histogrammes, des déplacements d'éléments et le sens de déplacement, dans les signaux issus des capteurs 7, 8. De plus, comme le moyen de perception dynamique 15 reçoit les signaux des deux capteurs, il est également capable d'identifier les déplacements d'éléments et le sens de déplacement, en trois dimensions.

Le moyen de perception structurelle 16 reçoit en entrée les signaux des deux capteurs 7, 8 et est configuré pour délivrer un ou des troisièmes signaux d'identification du geste, de préférence sous forme d'histogrammes spatio-temporels. Le moyen de perception structurelle 16 permet, pour différents signaux successifs, de détecter des formes et les bords orientés associés, et de les représenter sous forme d'histogrammes. Un tel dispositif et procédé est notamment décrit dans la demande de brevet FR 2 858 447.

Ainsi, le moyen de perception structurelle 16 permet d'identifier, à l'aide d'histogrammes, des formes et leurs orientations, dans les signaux issus des capteurs 7, 8. De plus, comme le moyen de perception structurelle 16 reçoit les signaux des deux capteurs, il est également capable d'identifier les formes et les orientations associées, en trois dimensions. La prise en compte des formes, notamment celles des avant-bras, et de leurs orientations permet d'identifier le geste du chirurgien, quelle que soit sa position autour du champ opératoire. Le dispositif de commande est ainsi apte à différencier :
- la commande associée à un mouvement effectué par un premier chirurgien vers la gauche, et
- la commande associée à un mouvement effectué par un second chirurgien faisant face au premier chirurgien, vers la droite,
les deux mouvements, qui correspondent à des commandes opposées, étant pourtant réalisés dans la même direction.

Les premiers, deuxièmes et troisièmes signaux d'identification du geste sont ensuite transmis au moyen de sélection de la commande 13. A partir des informations contenues dans les différents signaux d'identification du geste, le moyen de sélection de la commande 13 connaît l'élément commandant (la main du chirurgien) présent dans les signaux des capteurs 7, 8, et sa trajectoire en trois dimensions dans la zone commune de détection. A partir de cette trajectoire, et de gestes déterminés associés à des commandes mémorisées, le moyen de sélection de la commande 13 est apte à sélectionner la commande mémorisée correspondant au geste détecté. La commande ainsi sélectionnée est alors transmise, sous forme d'un signal de commande, au moyen de contrôle 11.

Les commandes associées aux gestes détectés peuvent être notamment paramétrées en fonction du chirurgien utilisant le système d'éclairage, ou en fonction du type d'opération chirurgicale effectuée. Ainsi, le moyen de sélection de la commande 13 peut être paramétré pour commander une seule propriété optique de l'ensemble de sources lumineuses 2, les différents gestes permettant de définir les différentes amplitudes de variations de ladite propriété optique. Alternativement, les propriétés optiques modifiables par le dispositif de commande 6 peuvent être modifiées en fonction du type d'opération.

Le moyen de sélection de la commande 13 peut comprendre, de préférence, un moyen d'activation 17. Le moyen d'activation 17 permet d'associer une commande à un geste détecté lorsque ce dernier remplit une condition déterminée. Par exemple, le moyen d'activation 17 peut autoriser une commande correspondant à un geste déterminé, uniquement si le geste a été effectué dans une partie définie de la zone commune de détection, par exemple à une distance déterminée de la surface éclairée. Ainsi, seuls les gestes effectués à une distance de, par exemple mais non exclusivement, cinquante centimètres de la surface éclairée, peuvent aboutir à une commande. Alternativement, le moyen d'activation 17 peut autoriser une commande uniquement si le geste est effectué avec une particularité déterminée, par exemple avec uniquement l'index et le pouce déployés.

Le moyen d'activation 17 permet ainsi de distinguer les gestes effectués pour commander le dispositif de commande 6 des gestes effectués par le chirurgien dans le cadre de l'invention.

Afin de permettre au chirurgien de savoir dans quelle mesure ses gestes sont pris en compte par le dispositif de commande 6, ce dernier peut comprendre un moyen d'information sonore 18 apte à émettre un signal sonore audible par le chirurgien. Le moyen d'information sonore 18 peut recevoir des signaux du moyen d'identification de la commande gestuelle, lorsqu'un geste est identifié comme correspondant à une commande ou bien lorsque le moyen d'activation 17 autorise une commande. Le moyen d'information sonore 18 peut également recevoir des signaux du moyen de contrôle 11, par exemple lorsqu'un signal de modification des propriétés optiques est envoyé. Ainsi, grâce au moyen d'information sonore 18, le chirurgien peut savoir dans quelle mesure ces instructions sont prises en compte par le dispositif de commande 6 ou bien si, par erreur, il a activé la reconnaissance gestuelle par un geste involontaire.

Par exemple, le moyen d'information sonore 18 peut émettre un signal sonore lorsque la main du chirurgien se situe à cinquante centimètres de la surface éclairée : le signal sonore indique au chirurgien que le moyen d'activation 17 du dispositif de commande 6 analyse ses gestes pour les traduire en commande. Puis, le moyen d'information sonore 18 peut émettre un signal sonore différent à chaque commande gestuelle détectée et effectuée par le dispositif de commande 6. Le chirurgien connaît alors exactement l'état et les actions effectuées par le dispositif de commande 6.

Ainsi, l'objet selon l'invention permet à un utilisateur de commander un système d'éclairage de manière précise et sans l'intervention d'un tiers, uniquement par des gestes effectués sans contact physique avec le système d'éclairage. L'objet de l'invention permet alors au chirurgien de commander le système d'éclairage sans le toucher, ce qui évite les problèmes d'aseptise durant l'opération. Par ailleurs, l'objet de l'invention reste souple d'utilisation en permettant une adaptation des commandes au chirurgien opérant ou au type d'opération effectuée. Enfin, grâce à un contrôle individuel ou groupé des sources lumineuses, il est également possible d'obtenir une modification des propriétés d'éclairage sans déplacer tout ou partie du système d'éclairage, permettant une réponse rapide à la commande gestuelle et un système d'éclairage plus fiable.

## Revendications

1. Système d'éclairage médical (1), notamment d'éclairage opératoire, comprenant :
- un ensemble de sources lumineuses (2) pour éclairer une surface déterminée et présentant une ou plusieurs propriétés optiques modifiables,
- un dispositif de commande (6) de l'ensemble de sources lumineuses (2), pour modifier ladite ou lesdites propriétés optiques en fonction d'une commande d'un utilisateur,
**caractérisé en ce que** le dispositif de commande (6) est un dispositif de commande à interface gestuelle comprenant :
- deux capteurs (7, 8) montés à distance l'un de l'autre, chaque capteur délivrant un signal correspondant à une zone de détection et les zones de détection des deux capteurs (7, 8) comprenant une zone de détection commune pour détecter une commande gestuelle de l'utilisateur, et
- un moyen d'analyse (9), recevant en entrée les signaux issus des deux capteurs (7, 8) et configuré pour modifier ladite ou lesdites propriétés optiques en fonction de ladite commande gestuelle lorsque la commande gestuelle est effectuée dans la zone de détection commune, à une distance de la surface déterminée supérieure à une valeur déterminée.

2. Système d'éclairage médical (1) selon la revendication 1 dans lequel le moyen d'analyse (9) comprend un moyen d'identification de la commande gestuelle (10) recevant en entrées les signaux issus des deux capteurs (7, 8) et configuré pour délivrer un signal de commande.

3. Système d'éclairage médical (1) selon la revendication 2 dans lequel le moyen d'identification de la commande gestuelle (10) comprend :
- un moyen de reconnaissance du geste (12) recevant en entrées les signaux issus des deux capteurs et configuré pour délivrer un ou des signaux identifiant un geste de l'utilisateur dans la zone commune des deux capteurs, et
- un moyen de sélection de la commande (13) recevant en entrée le ou les signaux délivrés par le moyen de reconnaissance du geste (12), et configuré pour délivrer le signal de commande correspondant au geste identifié par le moyen de reconnaissance du geste (12).

4. Système d'éclairage médical selon la revendication 3 dans lequel le moyen de reconnaissance du geste (12) comprend :
- un moyen de perception global (14) pour identifier les éléments présents dans la zone de détection commune, recevant en entrées les signaux des deux capteurs et configuré pour délivrer un ou des premiers signaux de reconnaissance du geste, de préférence sous forme d'histogrammes spatio-temporels,
- un moyen de perception dynamique (15) pour identifier des mouvements dans la zone de détection commune, recevant en entrées les signaux des deux capteurs et configuré pour délivrer un ou des deuxièmes signaux de reconnaissance du geste, de préférence sous forme d'histogrammes spatio-temporels, et
- un moyen de perception structurelle (16) pour identifier des formes dans la zone de détection commune, recevant en entrées les signaux des deux capteurs et configuré pour délivrer un ou des troisièmes signaux de reconnaissance du geste, de préférence sous forme d'histogrammes spatio-temporels.

5. Système d'éclairage médical selon la revendication 3 ou 4 dans lequel le moyen de sélection de la commande (13) comprend un moyen d'activation (17) configuré pour autoriser l'identification de la commande lorsque la commande gestuelle est effectuée dans la zone de détection commune, à une distance de la surface déterminée supérieure à une valeur déterminée.

6. Système d'éclairage médical selon l'une quelconque des revendications 2 à 5 dans lequel le moyen d'analyse (9) comprend un moyen de contrôle (11), recevant en entrée le signal de commande délivré par le moyen d'identification de la commande gestuelle (10) et configuré pour modifier ladite ou lesdites propriétés optiques en fonction dudit signal de commande.

7. Système d'éclairage médical (1) selon l'une quelconque des revendications 1 à 6 dans lequel le dispositif de commande (6) comprend également un moyen d'information sonore (18), pour émettre un signal sonore audible par l'utilisateur indiquant, par exemple, la détection d'une commande gestuelle ou l'attente d'une commande gestuelle.

8. Système d'éclairage médical (1) selon les revendications 5 et 7 dans lequel le moyen d'information sonore (18) est configuré pour émettre un signal sonore audible par l'utilisateur lorsque le moyen d'activation autorise l'identification de la commande.

9. Procédé de commande à interface gestuelle d'un système d'éclairage médical (1), notamment d'éclairage opératoire, le système d'éclairage médical (1) comprenant un ensemble de sources lumineuses (2) qui est configuré pour éclairer une surface déterminée et qui présente une ou plusieurs propriétés optiques modifiables, le procédé comportant les étapes successives suivantes :
a) une étape de détection, dans une zone de détection commune à deux zones de détection différentes, d'une commande gestuelle d'un utilisateur, puis
b) une étape d'analyse de ladite commande gestuelle afin de modifier ladite ou lesdites propriétés optiques en fonction de ladite commande gestuelle lorsque la commande gestuelle est effectuée dans la zone de détection commune, à une distance de la surface déterminée supérieure à une valeur déterminée.

10. Procédé de commande selon la revendication 9 dans lequel l'étape d'analyse comporte :
b1) une étape d'identification de ladite commande gestuelle.

11. Procédé de commande selon la revendication 10 dans lequel l'étape b1) d'identification de ladite commande gestuelle comprend :
b11) une étape de reconnaissance du geste, puis
b12) une étape de sélection de la commande correspondant au geste identifié à l'étape b11).

12. Procédé de commande selon la revendication 11 dans lequel l'étape b11) de reconnaissance du geste comprend :
- une perception globale identifiant les éléments présents dans la zone de détection commune,
- une perception dynamique identifiant des mouvements dans la zone de détection commune, et
- une perception structurelle identifiant des formes dans la zone de détection commune.

13. Procédé de commande selon la revendication 11 ou 12 dans lequel l'étape de sélection de la commande comprend une étape d'activation autorisant l'identification de la commande lorsque la commande gestuelle est effectuée dans la zone de détection commune, à une distance de la surface déterminée supérieure à une valeur déterminée.

14. Procédé de commande selon l'une quelconque des revendications 10 à 13 dans lequel l'étape d'analyse comprend une étape de modification de ladite ou desdites propriétés optiques en fonction de ladite commande gestuelle.

15. Procédé de commande selon l'une quelconque des revendications 9 à 14 comprenant également une étape d'information sonore durant laquelle un signal sonore audible par l'utilisateur est émis pour indiquer, par exemple, la détection d'une commande gestuelle ou l'attente d'une commande gestuelle.

16. Procédé de commande selon les revendications 13 et 15 dans lequel l'étape d'information sonore émet un signal sonore audible par l'utilisateur lorsque l'étape d'activation autorise l'identification de la commande.
